(19) Europäisches Patentamt / European Patent Office / Office européen des brevets

(11) **EP 4 058 588 B1**

(12) **EUROPEAN PATENT SPECIFICATION**

(45) Date of publication and mention of the grant of the patent:
**12.11.2025 Bulletin 2025/46**

(21) Application number: **20807380.9**

(22) Date of filing: **13.11.2020**

(51) International Patent Classification (IPC):
***C12N 15/87*** $^{(2006.01)}$ ***A61D 19/00*** $^{(2006.01)}$
***C12M 3/00*** $^{(2006.01)}$ ***C12M 1/42*** $^{(2006.01)}$

(52) Cooperative Patent Classification (CPC):
**C12N 15/87; C12M 21/06; C12M 35/00;** A61D 19/00

(86) International application number:
**PCT/EP2020/082142**

(87) International publication number:
**WO 2021/094588 (20.05.2021 Gazette 2021/20)**

# (54) USE OF PERFLUORO-N-OCTANE FOR PIEZO-MEDIATED INTRACYTOPLASMIC SPERM INJECTION

VERWENDUNG VON PERFLUOR-N-OCTAN FÜR DIE PIEZOVERMITTELTE INTRACYTOPLASMATISCHE SPERMAINJEKTION

UTILISATION DU PERFLUORO-N-OCTANE POUR L'INJECTION INTRACYTOPLASMIQUE DE SPERME À MÉDIATION PIÉZOÉLECTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.11.2019 GB 201916691**
**26.11.2019 GB 201917205**

(43) Date of publication of application:
**21.09.2022 Bulletin 2022/38**

(73) Proprietors:
- **Vitrolife Sweden Aktiebolag**
  **42132 Västra Frölunda (SE)**
- **Prime Tech Ltd**
  **Tsuchiura-shi, Ibaraki 300--0841 (JP)**

(72) Inventors:
- **PRIBENSZKY, Csaba**
  **42132 Västra Frölunda (SE)**
- **IWAMOTO, Masaki**
  **Tsuchiura-shi, Ibaraki 300--0841 (JP)**

(74) Representative: **D Young & Co LLP**
**3 Noble Street**
**London EC2V 7BQ (GB)**

(56) References cited:
**EP-A1- 3 467 118 WO-A1-2018/228872**

- **RUZZA PAOLO ET AL: "H-Content Is Not Predictive of Perfluorocarbon Ocular Endotamponade Cytotoxicity in Vitro", ACS OMEGA, vol. 4, no. 8, 20 August 2019 (2019-08-20), US, pages 13481 - 13487, XP093172736, ISSN: 2470-1343, Retrieved from the Internet <URL:https://www.ncbi.nlm.nih.gov/pmc/articles/PMC6705218/pdf/ao9b01793.pdf> DOI: 10.1021/acsomega.9b01793**
- **HIRAOKA KENICHIRO ET AL: "Clinical efficiency of Piezo-ICSI using micropipettes with a wall thickness of 0.625 [mu]m", JOURNAL OF ASSISTED REPRODUCTION AND GENETICS, PLENUM PUBLISHING, US, vol. 32, no. 12, 21 October 2015 (2015-10-21), pages 1827 - 1833, XP035912830, ISSN: 1058-0468, [retrieved on 20151021], DOI: 10.1007/S10815-015-0597-9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SALGADO R M ET AL: "Lower blastocyst quality after conventional vs. Piezo ICSI in the horse reflects delayed sperm component remodeling and oocyte activation", JOURNAL OF ASSISTED REPRODUCTION AND GENETICS, PLENUM PUBLISHING, US, vol. 35, no. 5, 10 April 2018 (2018-04-10), pages 825 - 840, XP036517548, ISSN: 1058-0468, [retrieved on 20180410], DOI: 10.1007/S10815-018-1174-9
- AKIRA HIRATA ET AL: "Use of an Ophthalmic Viscosurgical Device for Experimental Retinal Detachment in Rabbit Eyes", JOURNAL OF FUNCTIONAL BIOMATERIALS, vol. 4, no. 1, 18 January 2013 (2013-01-18), CH, pages 6 - 13, XP055766913, ISSN: 2079-4983, DOI: 10.3390/jfb4010006

## Description

FIELD OF THE INVENTION

The present invention relates to an apparatus and kit comprising purified perfluoro-n-octane or a composition comprising purified perfluoro-n-octane as operating liquid for Piezo-mediated intracytoplasmic sperm injection (Piezo-ICSI).

BACKGROUND TO THE INVENTION

Male factor infertility is responsible for the inability of approximately 50% of couples to conceive. Many procedures were developed in the 1980s to address fertilization failure due to dysfunctions of the male gamete including injecting a single sperm cell (spermatozoa) under the zona pellucida of the oocyte, with very limited success rates. However it was during the performance of subzonal injection of an oocyte that the oolemma was accidentally breached and the spermatozoon was delivered into the ooplasm, subsequently establishing the development of intracytoplasmic sperm injection as it is performed still today in humans.

Intracytoplasmic sperm injection (ICSI) is an *in vitro* fertilization procedure in which a sperm cell is injected directly into the cytoplasm of an oocyte. The first human pregnancy resulting from an embryo generated by ICSI was reported in 1992. ICSI is performed in a culture dish under a microscope by a human operator using micromanipulators and micropipettes to handle the oocyte and sperm. A fine glass micropipette (holding pipette) stabilizes a mature oocyte with gentle suction power applied by a microinjector whilst from the opposite side a thin, spiked glass micropipette (injection micropipette) is used to collect a single sperm, having immobilised it by cutting its tail with the point of the micropipette. Mechanical force applied by the operator through the micromanipulators is used to pierce the zona pellucida and plasma membrane (oolemma) of the oocyte with the injection micropipette. The sperm is then injected into the cytoplasm of the oocyte.

To date, ICSI has been responsible for over two million babies worldwide. However, ICSI is a labour-intensive technique, requiring a highly skilled operator, and there is still room for improvements with regards to standardisation of the technique and success rate. An alternative oocyte micro-injection method called "Piezo-mediated" ICSI (Piezo-ICSI) was developed for mouse oocytes, in 1995, as conventional ICSI was not solving the issues related to fertilisation in mouse *in vitro* fertilisation.

Piezo-ICSI resembles conventional ICSI (c-ICSI) in that a micropipette containing a single sperm pierces an oocyte to deliver the sperm to the oocyte cytoplasm. However, where in c-ICSI the zona pellucida and oolemma are pierced by mechanical force applied by the operator through micromanipulators to the micropipette, in Piezo-ICSI a piezoelectric effect is used to pierce the oocyte. The piezoelectric effect is the phenomenon that certain materials accumulate electric charge in response to mechanical stress, and conversely mechanical stress can be induced by application of electricity. The piezoelectric effect has many technological applications. In the case of Piezo-ICSI, a brief "Piezo pulse" is applied to the micropipette containing the sperm to produce ultra-fast, sub-micron forward momentum of the micropipette. This precise and rapid movement is used to pierce the zona pellucida and oolemma in a manner that is less stressful to the oocyte than the mechanical force used in c-ICSI.

The success rate of Piezo-ICSI was initially lower than c-ICSI. However, it was found that the success rate could be improved to be higher than that of c-ICSI by including a small amount of mercury in the injection micropipette.

Thus, Piezo-ICSI requires the presence of an "operating liquid" in the injection micropipette. At present, there is no complete scientific explanation of the physics of the piercing process in Piezo-ICSI. It has been suggested that the axial motion of the micropipette, induced by piezoelectricity, pierces the zona pellucida. It has also been hypothesized that the piercing is generated by the pressure burst caused by the abrupt forward motion of the operating liquid. It is also postulated that when the piezo pulse is applied, the micropipette moves forward but the operating liquid does not move due to the laws of inertia and the high specific gravity of the liquid. It is believed that the rapidly moving micropipette and stationary operating liquid generate negative pressure in the micropipette tip which pulls open the zona pellucida. More recent studies point out the occurrence of considerable lateral tip oscillations of the injection pipette as the piezoelectric pulse train is introduced. They claim that the lateral dynamics play an important role in the piercing and this process is also mediated by the operating liquid. Most probably, to some extent, all of the listed features take part in the effective operation of the piezo-mediated ICSI.

Although mercury is toxic and its usage extremely restricted, Piezo-ICSI is now used for *in vitro* fertilisation in animals. More recently, fluorinated compounds have been used as operating liquid.

Piezo-ICSI was first used successfully in mice in 1995 and later applied in porcine and bovine micro-injections. Piezo-ICSI achieved higher fertilization rates and survival rates than conventional ICSI (c-ICSI) and has therefore become the standard method in the art for micro-injection of sperm into animal oocytes.

Successful human pregnancy generated using the Piezo-ICSI technique was first reported in 1996 and improved

fertilization rates and survival rates were reported in 1999. However, Piezo-ICSI is not approved for clinical use in humans.

Further development of Piezo-ICSI to enable its approval for clinical use has the potential to improve the success rate of assisted reproduction in humans.

Salgado R M et al. 2018, J Assist. Reprod. Genet., 35(5) 825-840 relates to lower blastocyst quality after conventional vs. Piezo ICSI in the horse reflecting delayed sperm component remodelling and oocyte activation.

EP3467118A1 relates to a method for evaluating the cytotoxicity of chemical products.

## SUMMARY OF THE INVENTION

The invention relates to an operating liquid for Piezo-mediated microinjection.

The invention provides an apparatus for Piezo-mediated intracytoplasmic sperm injection (Piezo-ICSI) comprising an injection micropipette containing purified perfluoro-n-octane or a composition comprising purified perfluoro-n-octane as operating liquid, wherein the purified perfluoro-n-octane has an H-value of 1000 parts per million (ppm) or less.

The invention further provides a kit for Piezo-ICSI comprising purified perfluoro-n-octane or a composition comprising purified perfluoro-n-octane as operating liquid and one or more injection micropipettes, wherein the purified perfluoro-n-octane has an H-value of 1000 ppm or less.

Suitably, the use of purified perfluoro-n-octane as operating liquid as disclosed herein may provide advantages for methods of assisted reproduction. Such advantages may include higher proportion of successful fertilisation, improved rate of embryo development (e.g. higher proportion of embryos reaching the blastocyst stage) and higher proportion of successful implantation. The use of perfluoro-n-octane as operating liquid for Piezo-ICSI may be approved for clinical use in humans, providing the possibility to standardise ICSI procedures. Use of Piezo-ICSI may be a first step towards full automation of assisted reproduction. Piezo-ICSI is also a simpler technique to learn than c-ICSI, which requires a highly skilled operator. Use of perfluoro-n-octane as operating liquid for Piezo-ICSI thus has the potential to simplify and make more efficient methods of assisted reproduction in humans.

## DESCRIPTION OF THE FIGURES

**Figure 1 - Position of operating liquid in injection micropipette**

Figure 1 depicts an injection micropipette (1) into which operating liquid (2) has been loaded. The operating liquid forms a column roughly 10-15mm in length in the middle of the micropipette.

**Figure 2 - Attachment of micropipette to injector**

Figure 2A depicts insertion of an injection micropipette (1) into a micropipette holder (3).

Figure 2B depicts insertion of a micropipette holder (3) into an injector (4).

**Figure 3 - Piezo micromanipulator (PMM)/piezo driver**

Figure 3A depicts a piezo driver (5) attached to a micropipette holder (3).
Figure 3B is a photograph of a piezo driver (5).

**Figure 4** - **Injection micropipette and medium**

Figure 4A depicts media, such as PVP, being taken up by an injection micropipette.
Figure 4B is a photograph of an injection micropipette being used to take up PVP media.

**Figure 5** - **Schematic depiction of Piezo-ICSI**

Figure 5 depicts the steps of injection of a sperm into an oocyte using Piezo-ICSI. The tip of the injection micropipette is placed gently against the zona pellucida, without oocyte deformation (**a**). Piezo pulses are applied to the micropipette (**b**), which is advanced through the zona pellucida piercing a hole (**c**). The injection micropipette is withdrawn from the zona pellucida and the hollowed out piece of zona pellucida expelled by discharging the injection micropipette. This discharging is used to move the sperm to the tip of the injection micropipette (**d**). The injection micropipette is advanced through the zona pellucida (**e**) to roughly 80-90% across the diameter of the oocyte, pushing against and stretching the oolemma (**f**). One Piezo pulse is applied to break the oolemma, causing the cytoplasm to envelop the injection micropipette (**g**). The sperm is injected (**h**).

DETAILED DESCRIPTION

The invention provides an apparatus for Piezo-mediated intracytoplasmic sperm injection (Piezo-ICSI) comprising an injection micropipette containing purified perfluoro-n-octane or a composition comprising purified perfluoro-n-octane as operating liquid, wherein the purified perfluoro-n-octane has an H-value of 1000 parts per million (ppm) or less.

The invention further provides a kit for Piezo-ICSI comprising purified perfluoro-n-octane or a composition comprising purified perfluoro-n-octane as operating liquid and one or more injection micropipettes, wherein the purified perfluoro-n-octane has an H-value of 1000 ppm or less.

**Perfluoro-n-octane**

Perfluoro-n-octane consists of an unbranched chain of eight carbon atoms with each carbon atom bonded to the maximum number of fluorine atoms (i.e. three fluorine atoms bonded to the end-carbons, two fluorine atoms bonded to each other carbon). The IUPAC name of perfluoro-n-octane is 1,1,1,2,2,3,3,4,4,5,5,6,6,7,7,8,8,8-octadecafluorooctane. The CAS Registry Number of perfluoro-n-octane is 307-34-6. Perfluoro-n-octane has the chemical formula $C_8F_{18}$. The structural formula of perfluoro-n-octane is as follows:

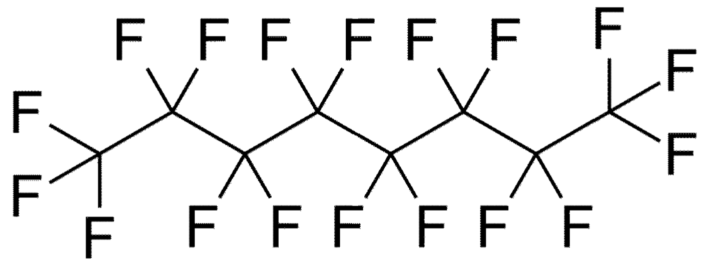

At room temperature, perfluoro-n-octane is an odourless, colourless liquid. Physical properties of perfluoro-n-octane are presented in Table 1 below (temperature dependent properties are quoted at 25 °C).

Table 1 - Physical properties of perfluoro-n-octane

| | |
|---|---|
| Boiling point | 103 °C |
| Pour point | -27 °C |
| Molecular weight | 438 |
| Density | 1.755 g/L |
| Dynamic viscosity | 1.03 $mm^2/s$ (1.8 mPa.s) |
| Vapour pressure | 3.4 mbar |
| Surface tension | 16 mN/m |

Perfluoro-n-octane is chemically inert. In other words, perfluoro-n-octane is stable and does not readily react with other compounds. This stability is due to the high strength of the carbon-fluorine bonds that predominate in its structure.

Perfluoro-n-octane may be referred to as a perfluorocarbon, a compound consisting solely of carbon and fluorine atoms. Perfluoro-n-octane may be referred to as a perfluorocarbon liquid (PFCL). Perfluoro-n-octane may be referred to as a perfluoroalkane, a compound consisting solely of carbon and fluorine atoms linked by single bonds.

Methods for synthesising perfluoro-n-octane are known in the art.

For example, perfluoro-n-octane may be synthesised by electrochemical fluorination, in which electrolysis is used to replace hydrogen atoms on an organic compound with fluorine atoms from a donor compound. Two illustrative forms of electrochemical fluorination that may be used are the Simons process and the Phillips Petroleum process. In the Simons process (Simons and Harland 1949 Journal of the Electrochemical Society 95: 47-66), an organic compound is electrolysed in a solution of hydrogen fluoride. The Phillips Petroleum process is similar to the Simons process, but uses potassium fluoride in hydrogen fluoride as a source of fluorine (Alsmeyer et al. 1994 Organofluorine Chemistry: Principles and Commercial Applications in Banks et al. (eds.). Organofluorine Chemistry. Boston, MA: Springer. pp. 121-143).

Perfluoro-n-octane may also be synthesised by the Fowler process (Fowler et al. 1947 Ind. Eng. Chem. 39: 292-298), wherein hydrocarbons are fluorinated in the vapour phase using cobalt(III) fluoride. In particular, in a first step, cobalt(II) fluoride is fluorinated to cobalt(III) fluoride by exposure to fluorine gas at high temperature. In a second step, the

cobalt(III) fluoride is exposed to the hydrocarbon to be fluorinated (e.g. octane, in the case of perfluoro-n-octane) at high temperature. The hydrogen atoms of the hydrocarbon are displaced by fluorine atoms from cobalt(III) fluoride, which is converted back to cobalt(II) fluoride.

Perfluoro-n-octane is readily available from commercial sources.

**Purified perfluoro-n-octane**

The term "purified perfluoro-n-octane" refers to perfluoro-n-octane that comprises low levels of underfluorinated impurities.

Underfluorinated impurities are by-products of the synthesis of perfluorocarbon compounds. Underfluorinated impurities comprise fewer fluorine atoms within their molecular structure than the intended perfluorocarbon product of the synthesis.

In particular, perfluoro-n-octane is completely fluorinated as each carbon atom in its molecular structure is bonded to the maximum number of fluorine atoms. However, during synthesis of perfluoro-n-octane, other molecules may additionally be produced that are incompletely fluorinated i.e. still contain hydrogen and/or carbon multiple bonds in the molecule and thus comprise fewer fluorine atoms than perfluoro-n-octane. These molecules are underfluorinated impurities.

Underfluorinated impurities of perfluoro-n-octane may comprise carbon-carbon double bonds. Underfluorinated impurities of perfluoro-n-octane may additionally or alternatively comprise carbon-carbon triple bonds. Underfluorinated impurities of perfluoro-n-octane may additionally or alternatively comprise one or more hydrogen atoms in place of the fluorine atoms found in perfluoro-n-octane.

Underfluorinated impurities may be referred to as underfluorinated compounds, underfluorinated by-products, incompletely fluorinated impurities, incompletely fluorinated compounds or incompletely fluorinated by-products.

In contrast to chemically inert perfluoro-n-octane, underfluorinated impurities are reactive. In other words, underfluorinated impurities can react with compounds that perfluoro-n-octane would not react with.

Underfluorinated impurities may be removed from synthesised perfluoro-n-octane by purification. In some embodiments of the present invention the purified perfluoro-n-octane comprises perfluoro-n-octane that has been subjected to purification.

Methods of purification of perfluorocarbon compounds, which may be applied to perfluoro-n-octane, are known in the art (see for example US3,696,156, US3,887,629 and US5,563,306). Such methods typically take advantage of the reactivity of underfluorinated impurities to distinguish them from the inert perfluoro-n-octane. In an exemplary method of purification of perfluoro-n-octane, underfluorinated impurities are reacted with an amine or a strong base, such as potassium hydroxide, in the presence of $Ca^{2+}$ or $Ba^{2+}$ ions.

The amount of underfluorinated impurities in a preparation of perfluoro-n-octane may be expressed as equivalent to the number of C-H bonds in these impurities. This value is termed the H-value and has units of parts per million (ppm). The amount of underfluorinated impurities in a preparation of perfluoro-n-octane may be measured using fluoride selective ionometry, for example.

The use of fluoride selective ionometry to quantify underfluorinated impurities is known in the art. The principle of the method is to react perfluoro-n-octane with a strong base that cleaves fluorine atoms from the molecules in the preparation, producing fluoride ions. The fluoride ions are then quantified, from which the amount of underfluorinated impurities may be calculated. An illustrative methodology for fluoride selective ionometry is set out below.

*Step 1: Chemical Transformation of Underfluorinated Impurities.*

Ten millilitres of the perfluorocarbon liquid (PFCL) are mixed with 3.4 g 1,6-diaminohexane and 15 mL nonane. This mixture is heated in a 100 mL glass flask equipped with a reflux condenser for 8 hours under stirring to a temperature of 120 °C. After cooling to room temperature, the solution is vigorously mixed with 30 mL hydrochloric acid (1.3 molar) and the aqueous phase is separated.

Subsequently, 15 mL of the aqueous phase are neutralized with 1.3 molar aqueous hydrochloric acid using phenolphthalein as an indicator and diluted to 25 mL with deionized water. Ten millilitres of this neutralized diluted solution are transferred into a 25 mL glass beaker and 1 mL TISAB-III is added under stirring.

*Step 2: Fluoride Selective Potentiometry*

Ion-selective potentiometry is used to quantify fluoride ions in the sample solution. Prior to sample measurement, calibration must occur using sodium fluoride solutions with fluoride ion concentrations between 0.005 mmol/L and 0.05 mmol/L as reference standard. In addition, a blank value is recorded. The sample solution is then analyzed. The amount of reactive underfluorinated impurities is expressed as equivalent to the number of C-H bonds in these impurities, the H-value. The H-value ($c_{F\text{-}C\text{-}H}$) is calculated using the following formula:

$$c_{F-C-H}[ppm] = \frac{1}{3} \times \left( \left( c_{F^-}\left[\frac{mmol}{L}\right] \times 5 \times \frac{\bar{M}_{PFCL}\left[\frac{g}{mol}\right]}{\rho_{PFCL}\left[\frac{g}{mL}\right]} \right) - bv[ppm] \right)$$

where:

$c_{F\text{-}C\text{-}H}$ is the concentration of incompletely fluorinated contaminants in the sample (i.e. the H-value);

$c_{F^-}$ is the measured concentration of fluoride ions in the sample;

$\overline{M}_{PFCL}$ is the molecular weight of the measured PFCL;

$\rho_{PFCL}$ is the density of the measured PFCL;

*bv* is the recorded blank value (reagent and boiling flask blank);

5 is the factor to compensate for the dilution steps; and

1/3 is the stoichiometric factor (calculation of number of CH bonds).

The purified perfluoro-n-octane has an H-value of about 1000 parts per million (ppm) or less. The purified perfluoro-n-octane may have an H-value of about 500 ppm or less. The purified perfluoro-n-octane may have an H-value of about 100 ppm or less. The purified perfluoro-n-octane may have an H-value of about 50 ppm or less. The purified perfluoro-n-octane may have an H-value of about 40 ppm or less. The purified perfluoro-n-octane may have an H-value of about 30 ppm or less. The purified perfluoro-n-octane may have an H-value of about 20 ppm or less. The purified perfluoro-n-octane may have an H-value of about 10 ppm or less. The purified perfluoro-n-octane may have an H-value of about 5 ppm or less. The purified perfluoro-n-octane may have an H-value of about 1 ppm or less. In a preferred embodiment the purified perfluoro-n-octane has an H-value of about 10 ppm or less.

The operating liquid has an H-value of about 1000 parts per million (ppm) or less. The operating liquid may have an H-value of about 500 ppm or less. The operating liquid may have an H-value of about 100 ppm or less. The operating liquid may have an H-value of about 50 ppm or less. The operating liquid may have an H-value of about 40 ppm or less. The operating liquid may have an H-value of about 30 ppm or less. The operating liquid may have an H-value of about 20 ppm or less. The operating liquid may have an H-value of about 10 ppm or less. The operating liquid may have an H-value of about 5 ppm or less. The operating liquid may have an H-value of about 1 ppm or less. In a preferred embodiment the operating liquid has an H-value of about 10 ppm or less.

Purified perfluoro-n-octane is available from commercial sources. In particular, purified perfluoro-n-octane is sold for use in vitreoretinal surgery. Examples of commercially available purified perfluoro-n-octane include PERFLUORON® Liquid sold by Alcon, BIO OCTANE™ PFS sold by Biotech and OPTISOL sold by Moss Vision Inc.

## Piezo-ICSI

Piezo-mediated intracytoplasmic sperm injection (Piezo-ICSI) is a technique known in the art for *in vitro* injection of sperm into the cytoplasm of an oocyte. Piezo-ICSI is useful for *in vitro* fertilization procedures in assisted reproduction. Piezo-mediated ICSI may also be referred to in the art as "Piezo-driven" ICSI or "Piezo-actuated" ICSI. Examples of use of Piezo-ICSI are provided by Kimura and Yanagimachi 1995 Biol. Reprod. 52(4): 709-720 and Hiraoka et al. 2019 Piezo-ICSI Chapter 39, pp 481-489 in Nagy et al. (eds) In Vitro Fertilization, Springer, Cham.

Piezo-ICSI is carried out on oocytes and sperm in a culture dish under a microscope. A human operator uses micromanipulators and micropipettes to handle the oocyte and sperm. A single oocyte is held in place by a holding micropipette via a gentle suction force whilst in the opposing micropipette, referred to as the "injection micropipette" to differentiate it from the micropipette holding the oocyte in place, a single sperm is captured. The injection micropipette is used to pierce first the zona pellucida of the oocyte and then the oolemma. A piezoelectric effect is used to pierce the zona pellucida and the oolemma. The piezoelectric effect is the phenomenon that certain materials accumulate electric charge

in response to mechanical stress, and conversely mechanical stress can be induced by application of electricity. In Piezo-ICSI, a brief "Piezo pulse" of electricity is applied to the injection micropipette containing the sperm to produce ultra-fast, sub-micron forward momentum of the injection micropipette. This precise and rapid movement is used to pierce the zona pellucida and oolemma. The sperm is then injected into the cytoplasm of the oocyte and the injection micropipette withdrawn from the oocyte.

The injection micropipette contains an operating liquid, which is required for safe penetration of the oocyte using the peizoelectric effect.

Thus Piezo-ICSI may comprise the following steps:

(a) providing an injection micropipette containing sperm and operating liquid;

(b) using Piezo pulses to pierce the oocyte with the injection micropipette; and

(c) injecting the sperm into the oocyte.

Step (b) may comprise:

(i) using Piezo pulses to pierce the zona pellucida of the oocyte;

(ii) moving the injection micropipette through the zona pellucida to push against the oolemma of the oocyte; and

(iii) using a Piezo pulse to pierce the oolemma and thereby moving the injection micropipette into the cytoplasm of the oocyte.

Accordingly, Piezo-ICSI may comprise the following steps:

(a) providing an injection micropipette containing sperm and operating liquid;

(b) using Piezo pulses to pierce the oocyte with the injection micropipette by:

  (i) using Piezo pulses to pierce the zona pellucida of the oocyte;

  (ii) moving the injection micropipette through the zona pellucida to push against the oolemma of the oocyte; and

  (iii) using a Piezo pulse to pierce the oolemma and thereby moving the injection micropipette into the cytoplasm of the oocyte; and

(c) injecting the sperm into the oocyte.

A "Piezo pulse" is the electricity applied to the injection micropipette to induce ultrafast submicron forward momentum of the micropipette. This momentum results from crystal deformation in response to the externally applied voltage. **In** addition to propelling the injection micropipette forward (axial momentum), lateral momentum is also generated. It is believed that both axial and lateral dynamics play important roles in piercing oocytes.

The equipment for carrying out Piezo-ICSI may comprise an inverted microscope (e.g. IX73) for viewing the oocyte, sperm and micropipettes, a three-axis micromanipulator (e.g. XenoWorksPT) for handling the oocyte, sperm and micropipettes, an injector (e.g. pneumatic microinjector such as PNJ-T2) for controlling injection through the injection micropipette and a Piezo micromanipulator (PMM), also termed a "piezo driver", (e.g. Piezo PMM4GD) for applying Piezo pulses to the injection micropipette. The consumables required for Piezo-ICSI may comprise a micropipette for injecting the sperm (e.g. Ultra-thin PINU06-20FT), a micropipette for holding the oocyte, operating liquid and a device for loading the operating liquid into the micropipette.

The invention provides an apparatus for Piezo-ICSI comprising an injection micropipette containing purified perfluoro-n-octane as operating liquid, wherein the purified perfluoro-n-octane has an H-value of 1000 parts per million (ppm) or less.

In some embodiments the apparatus for Piezo-ICSI according to the invention comprises an inverted microscope, a micromanipulator, an injector, an injection micropipette and a Piezo micromanipulator.

The invention also provides a consumables kit for Piezo-ICSI comprising purified perfluoro-n-octane as operating liquid and one or more injection micropipettes.

Suitably the consumables kit according to the invention comprises one or more holding pipettes.

Suitably the consumables kit according to the invention comprises a device for loading operating liquid into the one or more injection micropipettes.

Suitably the consumables kit according to the invention comprises purified perfluoro-n-octane as operating liquid, one or more injection micropipettes, one or more holding pipettes, and a device for loading operating liquid into the one or more injection micropipettes.

Piezo-ICSI may be carried out by following the steps set out below:

(a) Fill the injection micropipette with operating liquid using the loading device, to a length of about 2-25 mm, such as about 10-15 mm, within the micropipette (see Figure 1).

(b) Insert the injection micropipette into a micropipette holder (see Figure 2A) and attach this to the injector (see Figure 2B), connected to the Piezo micromanipulator (PMM) (see Figure 3).

(c) Position the injection micropipette under the microscope.

(d) Use the injector to push operating liquid to the head of the injection micropipette, so that no air remains.

(e) Move the tip of the micropipette into a droplet of medium containing the sperm. The medium may be nutritional medium or medium such as polyvinylpyrrolidone (PVP).

(f) Aspirate then discharge a small amount of medium from the droplet into the injection micropipette, ensuring not to completely aspirate or expel operating liquid. Repeat this until the interfacial boundary between the medium and the operating liquid inside the injection micropipette slides smoothly (see Figure 4).

(g) Immobilise a sperm cell and aspirate it into the injection micropipette. Sperm may be immobilised, for example, by crushing the tail with the micropipette tip. The sperm may be positioned within the injection micropipette roughly one oocyte diameter back from the injection micropipette tip.

(h) Position the oocyte using the holding micropipette. The oocyte should be positioned such that it can be pierced in the wide area of the pervitelline space, avoiding the spindle.

(i) Place the tip of the injection micropipette gently against the zona pellucida (see Figure 5**a**), without oocyte deformation, and turn on the PMM to apply Piezo pulses to the micropipette (see Figure 5**b**). Advance the injection micropipette through the zona pellucida piercing a hole by the ultrafast, submicron movements of the injection micropipette (see Figure 5**c**).

(j) Withdraw the injection micropipette from the zona pellucida and expel the hollowed out piece of zona pellucida remaining inside the injection micropipette by discharging the injection micropipette. Use this discharging of the injection micropipette to move the sperm to the tip of the injection micropipette (see Figure 5**d**).

(k) Set the PMM to single pulse setting. A single Piezo pulse is all that is required to break the oolemma.

(l) Advance the injection micropipette through the zona pellucida (see Figure 5**e**) to roughly 80-90% across the diameter of the oocyte, pushing against and stretching the oolemma (see Figure 5**f**).

(m) Activate the PMM to break the oolemma with one Piezo pulse. The cytoplasm envelops the injection micropipette (see Figure 5**g**).

(n) Inject the sperm, being careful to not discharge too much liquid into the cytoplasm (see Figure 5**h**).

(o) Withdraw the injection micropipette from the oocyte.

In c-ICSI an injection micropipette with a spiked or bevelled tip is used to pierce the oocyte with mechanical force. In Piezo-ICSI a flat-tipped injection micropipette may be used to pierce the oocyte (see Hiraoka et al. 2019 Piezo-ICSI Chapter 39, pp 481-489 in Nagy et al. (eds) In Vitro Fertilization, Springer, Cham.) as mechanical force is not used. Thus in one embodiment the injection micropipette is flat-tipped.

Injection micropipettes of different wall thickness have been used in Piezo-ICSI (Hiraoka and Kitamura 2015 J. Assist. Reprod. Genet. 32: 1827-1833). Injection micropipettes of any appropriate wall thickness may be used in the

methods of the invention. Suitably, the injection micropipette may have a wall thickness of between 0.5 μm and 1 μm. The injection micropipette may have a wall thickness of 0.925 μm. The injection micropipette may have a wall thickness of 0.625 μm.

The injection micropipette holding the sperm for Piezo-ICSI may also be referred to as a capillary or a microcapillary.

Piezo-ICSI may also comprise injection of a mammalian sperm into a mammalian oocyte. The sperm and oocyte may be human. The sperm and oocyte may be murine. The sperm and oocyte may be bovine. The sperm and oocyte may be porcine. The sperm and oocyte may be equine.

## Operating liquid

The term "operating liquid" refers to the liquid in the injection micropipette that assists piercing of the oocyte in Piezo-ICSI. If the micropipette does not contain operating liquid, but merely medium or oil, Piezo-mediated piercing of the oocyte is less efficacious and results in a higher ratio of oocyte degeneration. Therefore, use of operating liquid in the injection micropipette is advised to provide efficacious and safe piercing of the oocyte.

There is currently no scientific consensus on the precise physical effects of operating liquid that improve the results of Piezo-ICSI. Operating liquid has a relatively high specific gravity. It is believed that this high specific gravity means the operating liquid remains in place due to inertia when the injection micropipette moves. The stationary operating liquid may stabilize and suppress undesired lateral vibrations whilst intensifying axial (i.e. piercing) movement of the micropipette tip. The stationary operating liquid may also generate a slight vacuum as the micropipette moves axially, thereby creating slight suction that assists in opening the zona pellucida or oolemma.

The first operating liquid used was mercury. However, the toxicity of mercury requires it to be handled carefully in the laboratory, and it may not be used in Piezo-ICSI for humans.

Less toxic alternatives to mercury for use as operating liquids are fluorocarbons and fluoroethers.

In particular, fluorocarbon liquids, such as perfluoro n-alkyl morpholines (see Hiraoka et al. 2019 Piezo-ICSI Chapter 39, pp 481-489 in Nagy et al. (eds) In Vitro Fertilization, Springer, Cham.) and perfluorohydrocarbon mixtures, have been used as operating liquid in Piezo-ICSI. Fluorocarbon liquids are clear, colourless, odourless, non-flammable fluids having high specific gravity but a viscosity similar to water.

Commercially available hydrofluoroethers have also been used as operating liquid in Piezo-ICSI.

Though fluorocarbon liquids and hydrofluoroethers are used for Piezo-ICSI for animals, these compounds are not approved for use in human Piezo-ICSI. Furthermore, these liquids are not as efficacious as mercury for Piezo-ICSI.

The present disclosure relates to the use of purified perfluoro-n-octane or a composition comprising purified perfluoro-n-octane as operating liquid in Piezo-ICSI.

As described herein, the term "purified perfluoro-n-octane" means that the perfluoro-n-octane comprises low levels of reactive underfluorinated impurities. The operating liquid may thus comprise other compounds so long as these other compounds are substantially not underfluorinated impurities (i.e. underfluorinated compounds). In other words, the operating liquid may comprise other fully fluorinated compounds in addition to perfluoro-n-octane.

Thus, the operating liquid used in the present invention can be "purified perfluoro-n-octane" whilst comprising compounds other than perfluoro-n-octane because "purified" refers to the proportions of underfluorinated compounds in the operating liquid. The proportion of underfluorinated impurities is expressed by the H-value (ppm) as described herein.

The proportion of purified perfluoro-n-octane in the operating liquid may be expressed as a percentage. Likewise, the proportion of any other compound in the operating liquid may be expressed as a percentage. This percentage may refer to the percentage of the weight of the operating liquid which the purified perfluoro-n-octane or other compound comprises (wt.%) or the percentage of the volume of the operating liquid which the purified perfluoro-n-octane or other compound comprises (vol.%).

Suitably, the present use of purified perfluoro-n-octane as an operating liquid for Piezo-ICSI refers to operating liquid comprising about 90% or more purified perfluoro-n-octane, such as about 95% or more purified perfluoro-n-octane, such as about 96% or more purified perfluoro-n-octane, such as about 97% or more purified perfluoro-n-octane, such as about 98% or more purified perfluoro-n-octane, such as about 99% or more purified perfluoro-n-octane, such as 100% purified perfluoro-n-octane. In some embodiments the operating liquid comprises from about 95% to about 100% purified perfluoro-n-octane.

Preferably, the operating liquid comprises from about 95% to about 100% purified perfluoro-n-octane.

In some embodiments the operating liquid comprises about 95% or more perfluoro-n-octane and has an H-value of about 100 ppm or less. In some embodiments the operating liquid comprises about 95% or more perfluoro-n-octane and has an H-value of about 10 ppm or less.

The use of a composition comprising purified perfluoro-n-octane as an operating liquid for Piezo-ICSI is also disclosed herein.

Suitably, the use of a composition comprising purified perfluoro-n-octane as an operating liquid means that

purified perfluoro-n-octane should be the main component of the operating liquid (i.e. the compound forming the majority of the operating liquid). The composition used as the operating liquid may comprise other compounds in addition to purified perfluoro-n-octane.

For example, in some embodiments, the composition comprises purified perfluoro-n-octane as the main purified fully fluorinated compound. In other words, no other purified fully fluorinated compound is present in the composition at an amount equal to or greater than purified perfluoro-n-octane.

Suitably, the operating liquid may be a composition which comprises about 50% or more purified perfluoro-n-octane, such as about 60% or more purified perfluoro-n-octane, such as about 70% or more purified perfluoro-n-octane, such as about 80% or more purified perfluoro-n-octane, such as about 90% or more purified perfluoro-n-octane.

In some embodiments the operating liquid comprises other compounds in addition to perfluoro-n-octane. The other compounds are substantially not underfluorinated compounds. The other compounds are substantially fully fluorinated compounds.

The term "other fully fluorinated compounds" means fully fluorinated compounds other than perfluoro-n-octane.

In some embodiments the operating liquid comprises about 50% or less other fully fluorinated compounds, about 40% or less other fully fluorinated compounds, such as about 30% or less other fully fluorinated compounds, such as about 20% or less other fully fluorinated compounds, such as about 10% or less other fully fluorinated compounds, such as about 5% or less other fully fluorinated compounds, such as about 4% or less other fully fluorinated compounds, such as about 3% or less other fully fluorinated compounds, such as about 2% or less other fully fluorinated compounds, such as about 1% or less other fully fluorinated compounds, such as no other fully fluorinated compounds.

Perfluoro-n-heptane and perfluoro-n-nonane are examples of other fully fluorinated compounds that may be comprised in the operating liquid. In some embodiments the operating liquid comprises perfluoro-n-heptane. In some embodiments the operating liquid comprises perfluoro-n-nonane. In some embodiments the operating liquid comprises perfluoro-n-heptane and perfluoro-n-nonane. In some embodiments the operating liquid comprises perfluoro-n-heptane, perfluoro-n-nonane and other compounds.

Perfluoro-n-heptane and perfluoro-n-nonane each have the same structure as perfluoro-n-octane, except that instead of 8 carbon atoms they respectively comprise 7 carbon atoms and 9 carbon atoms. In other words, perfluoro-n-heptane consists of a chain of 7 carbon atoms wherein each carbon is bonded to the maximum number of fluorine atoms, and perfluoro-n-heptane consists of a chain of 9 carbon atoms wherein each carbon is bonded to the maximum number of fluorine atoms.

The structural formula of perfluoro-n-heptane is as follows:

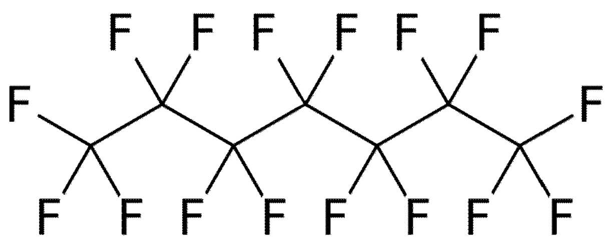

The structural formula of perfluoro-n-nonane is as follows:

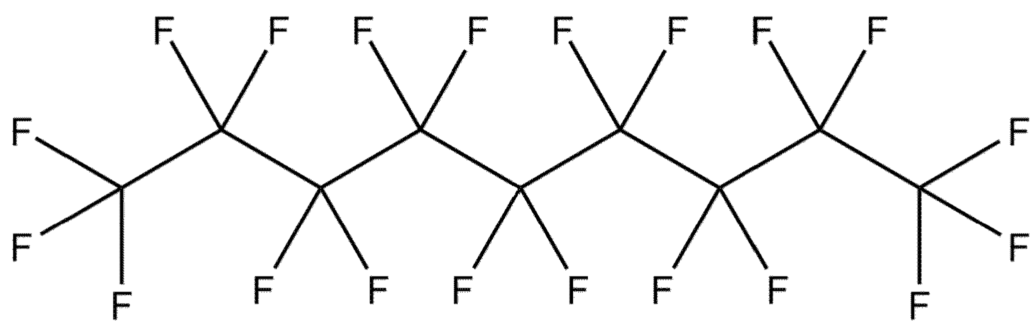

In some embodiments the operating liquid comprises about 5% or less perfluoro-n-heptane, such as about 4% or less perfluoro-n-heptane, such as about 3% or less perfluoro-n-heptane, such as about 2% or less perfluoro-n-heptane, such as about 1% or less perfluoro-n-heptane, such as no perfluoro-n-heptane.

In some embodiments the operating liquid comprises about 10% or less perfluoro-n-nonane, such as about 6% or less perfluoro-n-nonane, such as about 5% or less perfluoro-n-nonane, such as about 4% or less perfluoro-n-nonane,

such as about 3% or less perfluoro-n-nonane, such as about 2% or less perfluoro-n-nonane, such as about 1% or less perfluoro-n-nonane, such as no perfluoro-n-nonane.

In some embodiments the operating liquid comprises about 5% or less perfluoro-n-heptane and about 5% or less perfluoro-n-nonane. In some embodiments the operating liquid comprises about 2% or less perfluoro-n-heptane and about 5% or less perfluoro-n-nonane.

In some embodiments the operating liquid comprises from about 95 % to about 100% perfluoro-n-octane, about 2% or less perfluoro-n-heptane and about 5% or less perfluoro-n-nonane.

In some embodiments the operating liquid comprises from about 95 % to about 100% perfluoro-n-octane, about 2% or less perfluoro-n-heptane and about 5% or less perfluoro-n-nonane, and has an H-value of about 10 ppm or less.

**Assisted reproduction**

A method of assisted reproduction is disclosed comprising

(a) fertilising an oocyte *in vitro* to form an embryo;
(b) culturing the embryo; and
(c) implanting the embryo into a subject.

The subject may be human.

The method of assisted reproduction may comprise retrieving oocytes from a subject.

Techniques for assisted reproduction are known in the art. In particular, products and techniques for culturing embryos in vitro then implanting them in a subject are known in the art. For example, Vitrolife produces needles for oocyte retrieval (e.g. Sense oocyte retrieval needle), gradients for optimal sperm preparation (e.g. SpermGrad™), media for culturing fertilised oocytes and media for transferring viable and genetically sound embryos to a subject (e.g. EmbryoGlue).

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

Where a range of values is provided, each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the term "consisting of'.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

EXAMPLES

**Example 1 - Suitability of fluorinated compounds as operating liquid**

The suitability of three fluorinated test compounds for use as operating liquid for Piezo-ICSI was tested. The three test compounds were perfluorodecalin, perfluoro-n-octane and perfluoroperhydrophenanthrene. The test compounds were compared to a commercially available hydrofluoroether previously used as operating liquid as a control. The findings of this comparison are presented in Table 2 below.

Table 2 - Suitability for use of fluorinated compounds as operating liquids for Piezo-ICSI

| Operating liquid | Piezo driver settings | Evaluation of suitability for use as operating liquid for Piezo-ICSI |
|---|---|---|
| Commercially available hydrofluoroether (control) | Intensity = 2 Speed = 1 | **Good** - The control compound in this test has been used as operating liquid for a variety of routine clinical practices including sperm immobilization, zona pellucida opening and breaking oocyte membranes |
| Perfluorodecalin | Intensity = 4 Speed = 1 | **Poor** - Due to its high viscosity, it took more time to load perfluorodecalin into the ICSI pipette than the control operating liquid. For effective pulse transfer, the intensity of the piezo driver had to be increased to 4, or even higher when the injection pipette was loaded with extra volume of PVP. |
| Perfluoro-n-octane | Intensity = 2 Speed = 1 | **Good** - Loading of perfluoro-n-octane into the ICSI pipette was as easy as for the control operating liquid. Perfluoro-n-octane was very effective in piezo pulse transfer, with the same piezo driver settings used as for the control operating liquid. |
| Perfluoroperhydrophenanthrene | N/A | **Unusable** - The viscosity of perfluoroperhydrophenanthrene was very high, making it much harder to use than the other test compounds. It was extremely difficult to wash out excess perfluoroperhydrophenanthrene from inside or outside an injection micropipette. Since this characteristic might lead to damage the pipette holder of the ICSI setup, further testing of perfluoroperhydrophenanthrene was aborted after trying to load it into the injection micropipette. |

In summary, perfluoro-n-octane was found to be as suitable for use as operating liquid for piezo-ICSI as the standard hydrofluoroether operating liquid. Other fluorinated compounds were either less suitable or completely unsuitable.

**Example 2** - **Effects of fluorinated compounds on embryo development**

The effects of incubation in different fluorinated compounds on embryo development were tested.

223 2PN (two pronuclei) mouse embryos were randomly divided into four groups: A) raw perfluoro-n-octane; B) a commercially available perfluorohydrocarbon mixture previously used as operating liquid for Piezo-ICSI; C) purified perfluoro-n-octane; and D) untreated control.

Embryos in groups A, B and C were incubated for 3 minutes in the respective compound then transferred to culture medium. Embryos in all groups were then cultured for 5 days in an incubator (37.5 °C, relative 100% humidity, 5% $CO_2$ in air).

The number of embryos in each group showing signs of degeneration was counted.

The number of embryos in each group reaching the 2-cell, 3-cell, expanded blastocyst and hatching/hatched blastocyst stages of development within the 5-day incubation was counted.

The mean time taken to reach the 2-cell stage (T2) by those embryos that did, and the mean time taken for those embryos to develop from the 2-cell stage to the 3-cell stage (T3) were measured.

The results are presented in Table 3 below.

Table 3 - Mouse embryo development after incubation in fluorinated compounds

| Treatment group | # 2PN mouse embryos | # embryos showing degeneration | # 2-cell stage | # 3-cell stage | # expanded blastocyst | # hatching/ hatched blastocyst | Mean T2 (min) | Mean T2 to T3 (min) |
|---|---|---|---|---|---|---|---|---|
| A) raw perfluoro-n-octane | 55 | 0 | 52 (95%) | 45 (82%) | 36 (65%) | 9 (16%) | 1394 +/- 98 | 1518 +/-145 |

(continued)

| Treatment group | # 2PN mouse embryos | # embryos showing degeneration | # 2-cell stage | # 3-cell stage | # expanded blastocyst | # hatching/ hatched blastocyst | Mean T2 (min) | Mean T2 to T3 (min) |
|---|---|---|---|---|---|---|---|---|
| B) perfluor-ohy drocar-bon mixture | 57 | 4 | 45 (79%) | 39 (68%) | 33 (58%) | 2 (4%) | 1490 +/- 110 | 1670 +/-179 |
| D) purified perfluoro-n-octane | 55 | 0 | 51 (93%) | 48 (87%) | 45 (82%) | 33 (60%) | 1304 +/- 88 | 1293 +/-104 |
| D) untreated control | 56 | - | 52 (93%) | 50 (89%) | 47 (84%) | 32 (57%) | 1321 +/- 82 | 1307 +/-117 |

A small proportion of the embryos incubated in the commercially available perfluorohydrocarbon mixture operating liquid showed signs of degeneration, whereas none of the embryos incubated in perfluoro-n-octane showed signs of degeneration.

Embryos incubated in the commercially available perfluorohydrocarbon mixture operating liquid (group B) or raw perfluoro-n-octane (group C) developed more slowly than control embryos (group A). This slow-down is shown by the lower proportions of embryos reaching the expanded blastocyst and hatching/hatched blastocyst stages and the increased time taken to develop from the 2-cell stage to the 3-cell stage (mean T2 to T3).

In contrast, embryos incubated in purified perfluoro-n-octane (group D) developed at a pace comparable to untreated control embryos.

These data show that purified perfluoro-n-octane does not negatively affect embryo development, in contrast to the commercially available perfluorohydrocarbon mixture operating liquid.

**Example 3** - **Assisted reproduction using Piezo-ICSI with perfluoro-n-octane operating liquid**

The efficacy of assisted reproduction using c-ICSI was compared to using Piezo-ICSI was compared. Purified perfluoro-n-octane was used as operating liquid for Piezo-ICSI.

In particular, 69 patients having at least 6 mature oocytes were randomly divided into two groups. The first group underwent assisted reproduction in which sperm was introduced into oocytes using c-ICSI. The second group underwent assisted reproduction in which sperm was introduced into oocytes using Piezo-ICSI.

c-ICSI was performed using Eppendorf 2K control with ICSI micropipettes (TPC, Cooper Surgical). Piezo-ICSI was performed using PMM and Piezo micropipettes (Primetech). Purified perfluoro-n-octane was used as operating liquid in Piezo-ICSI.

Patients who underwent assisted reproduction in which Piezo-ICSI was used had significantly increased fertilization rates compared to patients who underwent assisted reproduction in which c-ICSI was used (80.6% of oocytes successfully fertilised using Piezo-ICSI vs 65.9% of oocytes successfully fertilised using c-ICSI, $p<0.05$). Using Piezo-ICSI also decreased oocyte degeneration rates compared to c-ICSI (3.8% using Piezo-ICSI vs 10.2% using c-ICSI).Utilisation rates were similar for c-ICSI and Piezo-ICSI (45.6% for c-ICSI vs 47.3% for Piezo-ICSI). Clinical pregnancy rates were also comparable for c-ICSI and Piezo-ICSI (50% for c-ICSI vs 55% for Piezo-ICSI).

Use of Piezo-ICSI produced on average 1 additional embryo for vitrification compared to c-ICSI (average of 3.8 embryos for vitrification from Piezo-ICSI vs average of 2.7 embryos for vitrification from c-ICSI).

These data show that use of Piezo-ICSI with purified perfluoro-n-octane as operating liquid in assisted reproduction is more efficacious than c-ICSI in producing embryos for assisted reproduction.

Various modifications and variations of the described methods and system of the invention will be apparent to those skilled in the art. Although the invention has been described in connection with specific preferred embodiments, it should be understood that the invention as claimed should not be unduly limited to such specific embodiments.

## Claims

1. Apparatus for Piezo-mediated intracytoplasmic sperm injection (Piezo-ICSI) comprising an injection micropipette containing purified perfluoro-n-octane or a composition comprising purified perfluoro-n-octane as operating liquid, wherein the purified perfluoro-n-octane has an H-value of 1000 parts per million (ppm) or less.

2. The apparatus according to claim 1, wherein the apparatus comprises an inverted microscope, a micromanipulator, an injector, an injection micropipette and a Piezo micromanipulator.

3. A kit for Piezo-ICSI comprising purified perfluoro-n-octane or a composition comprising purified perfluoro-n-octane as operating liquid and one or more injection micropipettes, wherein the purified perfluoro-n-octane has an H-value of 1000 ppm or less.

4. The kit according to claim 3 further comprising one or more holding pipettes.

5. The kit according to claim 3 or claim 4 further comprising a device for loading the operating liquid into the one or more injection micropipettes.

6. The apparatus according to claim 1 or claim 2, or the kit according to any of claims 3 to 5, wherein the purified perfluoro-n-octane has an H-value of 10 ppm or less.

7. The apparatus according to any of claims 1, 2 and 6, or the kit according to any of claims 3 to 6, wherein the composition comprises 90% or more purified perfluoro-n-octane.

8. The apparatus or kit according to claim 7, wherein the composition comprises 95% or more purified perfluoro-n-octane.

## Patentansprüche

1. Vorrichtung zur Piezo-vermittelten intracytoplasmatischen Spermieninjektion (Piezo-ICSI), umfassend eine Injektionsmikropipette, die gereinigtes Perfluor-n-octan oder eine gereinigtes Perfluor-n-octan umfassende Zusammensetzung als Betriebsflüssigkeit enthält, wobei das gereinigte Perfluor-n-octan einen H-Wert von 1000 ppm (parts per million) oder kleiner aufweist.

2. Vorrichtung nach Anspruch 1, wobei die Vorrichtung ein inverses Mikroskop, einen Mikromanipulator, einen Injektor, eine Injektionsmikropipette und einen Piezo-Mikromanipulator umfasst.

3. Kit für Piezo-ICSI, umfassend gereinigtes Perfluor-n-octan oder eine gereinigtes Perfluor-n-octan umfassende Zusammensetzung als Betriebsflüssigkeit und ein oder mehrere Injektionsmikropipetten, wobei das gereinigte Perfluor-n-octan einen H-Wert von 1000 ppm oder kleiner aufweist.

4. Kit nach Anspruch 3, ferner umfassend ein oder mehrere Haltepipetten.

5. Kit nach Anspruch 3 oder Anspruch 4, ferner umfassend ein Gerät zum Laden der Betriebsflüssigkeit in die eine oder mehreren Injektionsmikropipetten.

6. Vorrichtung nach Anspruch 1 oder Anspruch 2 oder Kit nach einem der Ansprüche 3 bis 5, wobei das gereinigte Perfluor-n-octan einen H-Wert von 10 ppm oder kleiner aufweist.

7. Vorrichtung nach einem der Ansprüche 1, 2 und 6 oder Kit nach einem der Ansprüche 3 bis 6, wobei die Zusammensetzung 90 % oder mehr gereinigtes Perfluor-n-octan umfasst.

8. Vorrichtung oder Kit nach Anspruch 7, wobei die Zusammensetzung 95 % oder mehr gereinigtes Perfluor-n-octan umfasst.

## Revendications

1. Appareil pour l’injection intracytoplasmique de sperme à médiation piézoélectrique (Piézo-ICSI) comprenant une micropipette d’injection contenant du perfluoro-n-octane purifié ou une composition comprenant du perfluoro-n-octane purifié comme liquide de fonctionnement, dans lequel le perfluoro-n-octane purifié a une valeur H de 1 000 parties par million (ppm) ou moins.

2. Appareil selon la revendication 1, dans lequel l'appareil comprend un microscope inversé, un micromanipulateur, un injecteur, une micropipette d'injection et un micromanipulateur piézo.

3. Kit pour Piézo-ICSI comprenant du perfluoro-n-octane purifié ou une composition comprenant du perfluoro-n-octane purifié comme liquide de fonctionnement et une ou plusieurs micropipettes d'injection, dans lequel le perfluoro-n-octane purifié a une valeur H de 1 000 ppm ou moins.

4. Kit selon la revendication 3, comprenant en outre une ou plusieurs pipettes de maintien.

5. Kit selon la revendication 3 ou la revendication 4, comprenant en outre un dispositif pour charger le liquide de fonctionnement dans la ou les micropipettes d'injection.

6. Appareil selon la revendication 1 ou la revendication 2, ou kit selon l'une quelconque des revendications 3 à 5, dans lequel le perfluoro-n-octane purifié a une valeur H de 10 ppm ou moins.

7. Appareil selon l'une quelconque des revendications 1, 2 et 6, ou kit selon l'une quelconque des revendications 3 à 6, dans lequel la composition comprend 90 % ou plus de perfluoro-n-octane purifié.

8. Appareil ou kit selon la revendication 7, dans lequel la composition comprend 95 % ou plus de perfluoro-n-octane purifié.

Figure 1

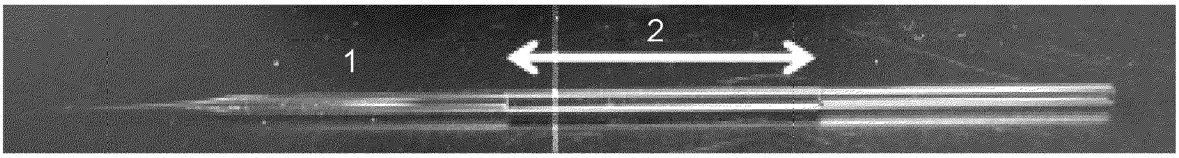

Figure 2

A

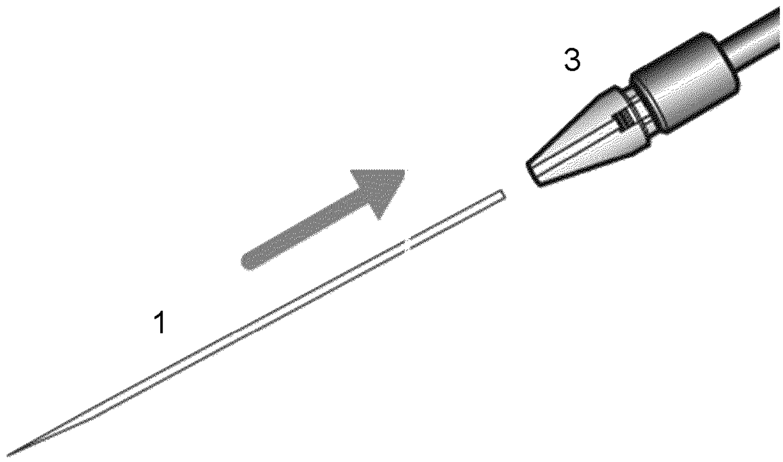


B

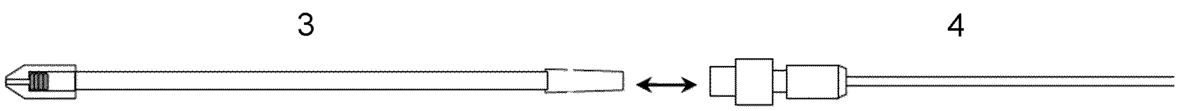

Figure 3

A

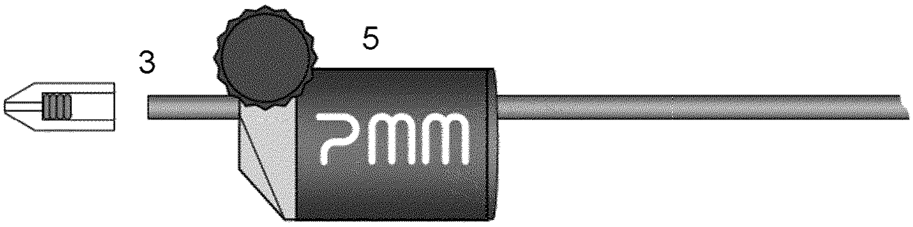


B

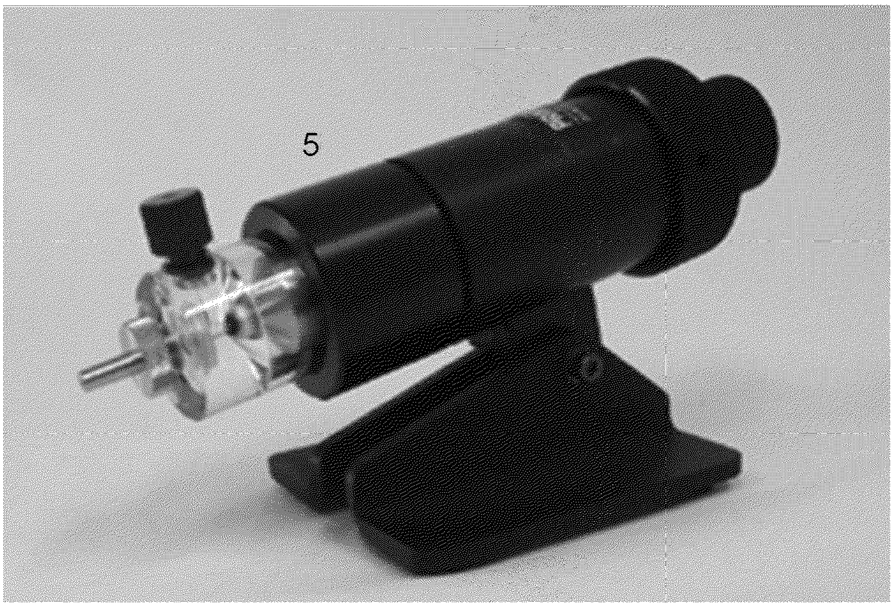

Figure 4

A

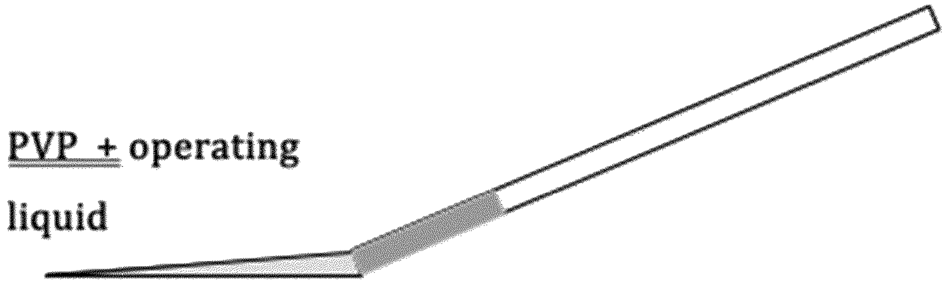


B

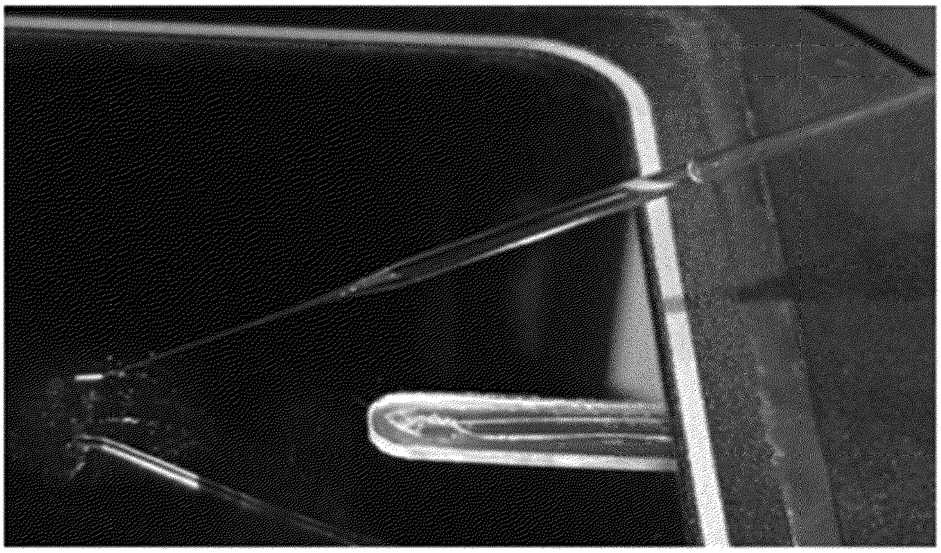

Figure 5

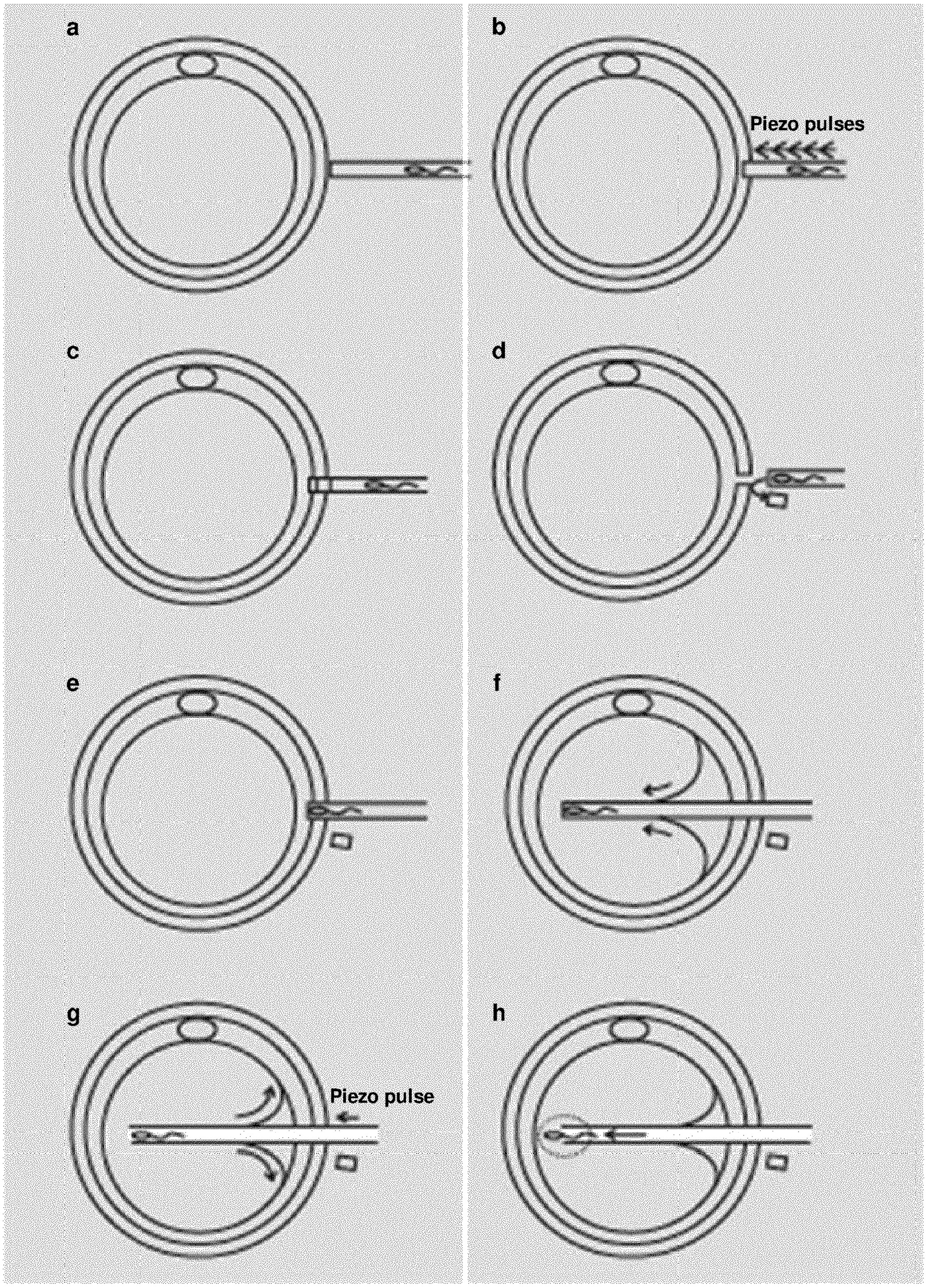

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3467118 A1
- US 3696156 A
- US 3887629 A
- US 5563306 A


### Non-patent literature cited in the description


- **SALGADO R M et al.** *J Assist. Reprod. Genet.*, 2018, vol. 35 (5), 825-840
- *CHEMICAL ABSTRACTS*, 307-34-6
- **SIMONS** ; **HARLAND**. *Journal of the Electrochemical Society*, 1949, vol. 95, 47-66
- Organofluorine Chemistry: Principles and Commercial Applications. **ALSMEYER et al.** Organofluorine Chemistry. Springer, 1994, 121-143
- **FOWLER et al.** *Ind. Eng. Chem.*, 1947, vol. 39, 292-298
- **KIMURA** ; **YANAGIMACHI**. *Biol. Reprod.*, 1995, vol. 52 (4), 709-720
- Piezo-ICSI. **HIRAOKA et al.** Vitro Fertilization. Springer, Cham, 2019, 481-489
- **HIRAOKA** ; **KITAMURA**. *J. Assist. Reprod. Genet.*, 2015, vol. 32, 1827-1833
- Piezo-ICSI. **HIRAOKA et al.** Vitro Fertilization. Springer, Cham, 2019, vol. 39, 481-489